# DEMANDE DE BREVET EUROPEEN

(11) **EP 0 645 138 A2**
(43) Date de publication de la demande: **29.03.1995**
(21) Numéro de dépôt: 94402032.0
(22) Date de dépôt: 13.09.1994
(51) Int. Cl.: A61K 31/195, A61K 31/44

(54) **Composition pharmaceutique visant à maintenir et/ou rétablir un taux d'agrégation des plaquettes sanguines proche de la valeur normale**

(30) Priorité: 14.09.1993 FR 9310925
(71) Demandeur: Dehorne, Marthe, F-44240 La Chapelle sur Erdre (FR)
(72) Inventeur: Dehorne, Marthe, F-44240 La Chapelle sur Erdre (FR)
(74) Mandataire: Keib, Gérard

(57) **Abrégé**

L'invention concerne une composition pharmaceutique administrable à l'homme ou à l'animal destinée à maintenir et/ou à rétablir un taux d'agrégation des plaquettes sanguines proche de la normale, caractérisée en ce qu'elle comprend de la cystéine et/ou de la cystine, et/ou leurs dérivés, à une dose et sous une forme physiologiquement acceptables.

## Description

L'invention a pour objet une composition pharmaceutique administrable à l'homme ou à l'animal qui vise à maintenir et/ou à rétablir un taux d'agrégation des plaquettes sanguines proche de la valeur normale.

Elle a également pour objet l'utilisation d'une composition pour la préparation d'un médicament administrable à l'homme ou à l'animal visant à maintenir et/ou à rétablir un taux d'agrégation des plaquettes sanguines proche de la valeur normale.

Il est connu depuis longtemps que les plaquettes sanguines présentes dans toutes les espèces du règne animal ont pour fonction principale d'empêcher toute sortie de sang à l'extérieur de l'espace vasculaire ou de l'organisme.

Cette sortie de sang résulte de la lésion d'un ou de plusieurs vaisseaux et est due à différents facteurs:
- naturels, par exemple des lésions ou micro lésions des vaisseaux qui se produisent tout au long de la vie d'un organisme,
- accidentels, par exemple à l'occasion d'un acte chirurgical, d'un traumatisme, d'une maladie ou d'un traitement médicamenteux.

Dès qu'une lésion apparaît dans un vaisseau, les plaquettes sanguines adhèrent à la paroi lésée en se regroupant entre elles. C'est le phénomène appelé d'agrégation plaquettaire. De cette façon, les plaquettes forment un "bouchon" à l'endroit de la lésion, stoppant l'hémorragie jusqu'à ce que la cicatrisation du vaisseau soit achevée.

Généralement, cette agrégation plaquettaire prend place rapidement après la formation d'une lésion.

Les saignements sont alors limités dans le temps et les risques d'hémorragie plus importante restent faibles.

Il arrive cependant que les plaquettes sanguines présentent des capacités d'agrégation limitées, voire inexistantes.

Les hémorragies ne sont alors stoppées que lentement, avec les inconvénients qu'elles entraînent pour l'individu, notamment difficultés de récupération de la forme physique, nécessité de transfusion, risques d'infection. Dans le cas extrême où les plaquettes sanguines ont perdu totalement leur capacité d'agrégation, I'hémorragie ne peut plus être stoppée, conduisant le plus souvent à la mort de l'individu.

Ces dysfonctionnements des plaquettes sanguines sont généralement liés à une baisse importante de leur nombre (thrombopénie), ou à une perte de leur capacité fonctionnelle (thrombopathie).

Ils se rencontrent dans un certain nombre de maladies infectieuses liées à des virus, à des bactéries ou à des parasites, notamment les infections rhinopharyngées, la rougeole, la varicelle, les oreillons, les hépatites ; dans certaines maladies générales appelées connectivites; dans certaines maladies thyroïdiennes, par exemple la maladie de Basedow et la thyroïdite de Hashimoto ; dans les maladies hématologiques telles que leucémies et lymphomes ; dans certains cancers et lors de certains traitements médicamenteux.

Pour ce qui concerne les traitements médicamenteux, il a été démontré en effet que certains médicaments sont responsables, à titre d'effets secondaires, de thrombopénies et dans certains cas de thrombopathies.

Une liste non limitative de ces médicaments sera donnée plus loin.

Il existe donc toujours à l'heure actuelle un besoin en une substance capable de minimiser les risques d'altération majeure de la capacité d'agrégation des plaquettes sanguines, liés notamment à l'utilisation de certains médicaments.

Le but de la présente invention est de répondre en grande partie à cette attente, en proposant une composition pharmaceutique possédant une action favorable sur le taux d'agrégation plaquettaire.

La composition pharmaceutique selon l'invention, administrable à l'homme ou à l'animal, destinée à maintenir et/ou rétablir un taux d'agrégation des plaquettes sanguines proche de la valeur normale, se caractérise en ce qu'elle comprend, à titre de principe actif, de la cystéine et/ou de la cystine et/ou leurs dérivés, à une dose et sous une forme physiologiquement acceptables.

Selon un second objet de la présente invention, l'invention utilise pour la préparation d'un médicament administrable à l'homme ou à l'animal, destiné à maintenir ou à rétablir un taux d'agrégation des plaquettes sanguines proche de la valeur normale, une composition comprenant de la cystéine et/ou de la cystine, et/ou leurs dérivés, ladite composition étant présente à une dose et sous une forme physiologiquement acceptables.

Les inventeurs ont en effet découvert, de façon surprenante et totalement inattendue, que la cystéine et ses dérivés, jusqu'à présent connus pour leurs propriétés désintoxiquantes, mucolytiques et asséchantes ainsi que la cystine et ses dérivés, connus pour leur action sur la croissance des cheveux et des ongles, étaient également capables de restituer ou de maintenir un taux d'agrégation des plaquettes sanguines au collagène et à la ristocétine proche de la valeur normale, tout en maintenant une hypoaggrégabilité souhaitable à l'acide arachidonique.

Selon une forme de réalisation de l'invention, la composition pharmaceutique selon l'invention comprend de la cystéine ou de la cystine, sous leur forme naturelle. Selon une autre forme de réalisation de l'invention, la composition selon l'invention comprend de la cystéine ou de la cystine, sous forme d'un sel. On citera à titre d'exemples non limitatifs le sel de sélénium de la cystéine (sélénocystéine), les sels de métaux endogènes de la cystéine ou de la cystine, tels que sels de sodium, calcium, magnésium ou potassium.

La salification peut également être effectuée par action d'une base organique, telles que la triéthylamine, la propylamine, la N,N-diméthyléthanolamine, le tris (hydroxyméthyl) amino méthane, l'éthanolamine, la pyridine et la morpholine.

Selon une autre forme de réalisation de l'invention, la composition selon l'invention comprend au moins un dérivé de la cystéine ou de la cystine.

Des exemples préférés de dérivés de la cystéine sont notamment l'acétylcystéine, la méthylcystéine, la carboxyméthylcystéine, I'éthylcystéine, la dansylcystéine, la carboxyéthylcystéine.

Des exemples préférés de dérivés de la cystine sont notamment la dansylcystine, la dinitrophénylcystine.

Les dérivés ci-dessus peuvent être présents sous leur forme naturelle ou salifiée tel que cela a été décrit plus haut.

Selon une autre forme de réalisation, la composition selon l'invention comprend de la cystéine et de la cystine, et/ou leurs dérivés sous forme de mélange.

Avantageusement, la composition selon l'invention comprend une dose de principe actif variant entre environ 30 mg et environ 4 000 mg, de préférence entre environ 100 mg et environ 1 000 mg.

De préférence, la composition selon l'invention comprend de plus de la vitamine B₆, en association avec le principe actif.

L'invention trouve une application particulièrement préférée en tant que médicament associé à un traitement utilisant des médicaments responsables ou soupçonnés responsables d'induire à titre d'effets secondaires des thrombopénies ou thrombopathies.

En particulier, l'invention est tout à fait adaptée comme traitement associé à la prise de médicaments à base de paracétamol, d'inhibiteur calcique, d'acétazolamide, d'acide acétylsalicylique, d'amidopyrine, d'apronalide, de carbamazépine, de chloropropamide, de chlorothiazide, de cimétidine, de digitoxine, de diphénylhydantoïne, d'héparine, de quinidine ou de quinine.

La liste de médicaments ci-dessus n'a bien évidemment aucun caractère limitatif. D'autres principes actifs, pour lesquels une action altérante du fonctionnement des plaquettes sanguines serait mise en évidence, pourraient être avantageusement utilisés avec la composition objet de la présente invention.

Dans l'utilisation préférée de l'invention comme traitement associé à une composition pharmaceutique responsable ou soupçonnée responsable d'altération du fonctionnement des plaquettes sanguines, la composition de l'invention peut être administrée séparément de la composition pharmaceutique, sous l'une des formes qui vont être décrites dans ce qui suit.

Selon une autre forme de réalisation de l'invention, la composition est directement incluse dans la composition pharmaceutique responsable ou soupçonnée responsable d'altération du fonctionnement des plaquettes, en tant qu'ingrédient supplémentaire.

La dose administrée est variable selon le degré de l'affection traitée, le sujet en cause, la voie d'administration et le produit considéré.

Elle peut être comprise, par exemple, entre environ 0,1 g et environ 5 g par jour. Généralement, les doses journalières sont réparties en deux ou trois prises par journée.

La composition selon l'invention peut être administrée par voie orale, par voie parentérale, notamment intramusculaire, intraveineuse ou sous-cutanée, ou par voie rectale.

Pour l'administration orale, la composition de l'invention peut se présenter sous forme de comprimé, pilule, poudre, granulé, sirop.

Pour l'administration par voie parentérale, la composition selon l'invention peut se présenter sous la forme de solution stérile aqueuse, de suspension ou d'émulsion.

Pour l'administration par voie rectale, la composition selon l'invention peut se présenter sous la forme de suppositoire.

Lorsque la composition selon l'invention est administrée en association avec un traitement responsable de l'altération des plaquettes, les deux traitements sont généralement poursuivis conjointement.

Dans d'autres cas, en particulier lorsque la composition de l'invention est administrée seule, la durée du traitement dépend généralement d'un retour du taux d'agrégation des plaquettes à une valeur proche de la normale.

Il appartiendra dans tous les cas à l'homme du métier de vérifier, par des analyses effectuées à intervalles réguliers, que le taux d'agrégation des plaquettes se situe à des valeurs acceptables pour pouvoir, le cas échéant, modifier les doses ou interrompre le traitement.

La composition selon l'invention peut également contenir des excipients habituellement utilisés dans l'industrie pharmaceutique, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants, émulsifiants ou aromatisants, les conservateurs.

Des caractéristiques et avantages supplémentaires de la présente invention apparaîtront encore à la lumière de la description plus détaillée qui suit d'exemples de réalisation de l'invention donnés à titre illustratif, ainsi qu'aux figures qui s'y rapportent et dans lesquelles :
- la figure 1 représente les variations du taux d'agrégation des plaquettes sanguines obtenues par la mise en oeuvre de l'invention avec la cystéine et la cystine,
- la figure 2 représente les variations du taux d'agrégation des plaquettes sanguines obtenues par la mise en oeuvre de l'invention avec la cystine.

Dans ce qui suit, le taux d'agrégation plaquettaire est déterminé par réaction en présence de collagène, d'adrénaline, d'adénosine diphosphate, d'acide arachidonique ou de ristocétine, selon la méthode décrite dans BORN V.R. J.Physiol. 1963, 168, 178 et O'BRIEN JR., J. Clin. Path. (London) 1962, 15, 452.

L'appareil utilisé est un lumiagrégomètre Chronolog (COULTRONICS, France). Les réactifs utilisés sont disponibles commercialement auprès de la société SIGMA, France.

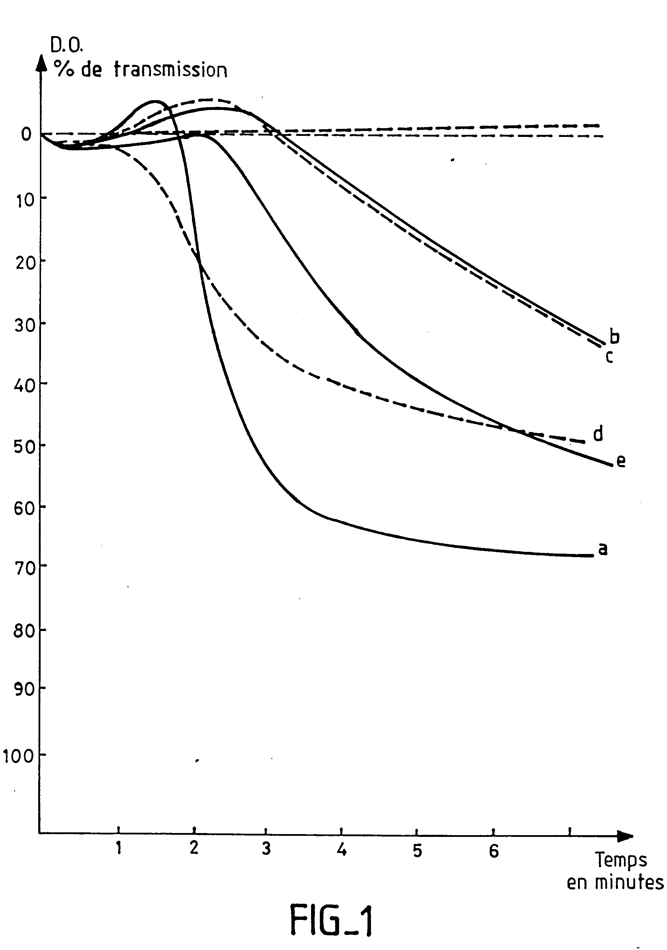

La figure 1 représente les effets sur le taux d'agrégation plaquettaire de la cystéine et de la cystine, testées sur un malade, ci-après appelé malade A. La courbe a représente le taux d'agrégation normal du malade A, sans prise de médicaments.

Les courbes b et c représentent les taux d'agrégation in vitro et in vivo respectivement du malade A, obtenus après traitement par acide acétylsalicylique. L'acide acétylsalicylique est administré sous forme d'ASPEGIC® disponible commercialement auprès des Laboratoires SYNTHELABO France, pendant une durée de 8 jours.

Il apparaît clairement sur les deux courbes b et c que l'agrégation est largement perturbée.

On administre alors au malade A, en association avec l'acide acétylsalicylique, de la cystine à la dose de 3 g par jour.

La cystine est administrée sous forme de comprimés de CYSTINE B6 BAILLEUL®, disponibles commercialement auprès des Laboratoires BAILLEUL France.

Les résultats obtenus après quatre jours de traitement figurent sur la courbe e qui représente le taux d'agrégation in vivo du malade A sous traitement avec l'acide acétylsalicylique et la cystine. Parallèlement, le taux d'agrégation du malade A est également mesuré in vitro par ajout de cystéine (disponible commercialement auprès de la société SIGMA France), à une dose équivalente à celle utilisée avec la cystine in vivo. Le résultat obtenu est représenté sur la courbe d. Il apparaît dans les deux cas que le taux d'agrégation est nettement amélioré et tend à revenir vers une valeur normale.

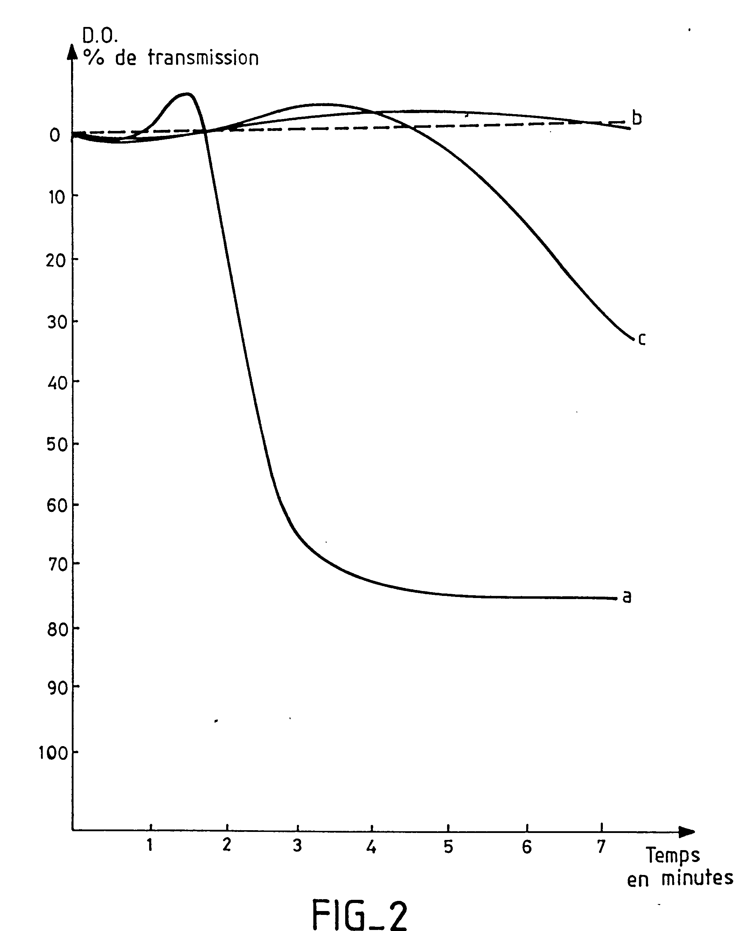

La figure 2 représente les résultats obtenus avec la cystine, testée sur un malade B.

La courbe a représente le taux d'agrégation du malade B, sous l'effet d'aucun traitement médicamenteux.

La courbe b représente le taux d'agrégation in vivo du malade B, sous traitement à l'acide acétylsalicylique administré de la même façon que précédemment.

Il apparaît nettement que le malade B n'agrège plus du tout.

Le malade B reçoit en association avec l'acide acétylsalicylique de la cystine, à la dose de 3 g par jour. La cystine est administrée sous forme de comprimés de CYSTINE B6 BAILLEUL®, disponibles commercialement auprès des Laboratoires BAILLEUL France.

Le résultat obtenu in vivo est représenté sur la courbe c, après 8 jours de traitement.

Il apparaît que l'administration de cystine en association avec l'acide acétylsalicylique tend à faire revenir le taux d'agrégation à un niveau acceptable, proche de la normale.

Il va de soi que l'invention ne se limite nullement aux formes de réalisation particulières qui ont été décrites dans ce qui précède, mais en embrasse au contraire toutes les variantes.

Une variante possible de l'invention consiste par exemple en l'utilisation de doses plus ou moins élevées que celles décrites dans ce qui précède, en fonction de durées de traitement différentes ou de cas particuliers. L'homme de métier aura toute liberté d'adapter la mise en oeuvre de l'invention par de simples opérations de mise au point, sans pour autant sortir du cadre de l'invention.

Une seconde variante de l'invention consiste dans le choix de dérivés de la cystéine ou de la cystine différents de ceux indiqués dans ce qui précède.

## Revendications

1. Utilisation pour la préparation d'un médicament administrable à l'homme ou à l'animal destiné à maintenir et/ou à rétablir un taux d'agrégation des plaquettes sanguines proche de la valeur normale, d'une composition comprenant, à titre de principe actif, de la cystéine et/ou de la cystine, et/ou leurs dérivés, à une dose et sous une forme physiologiquement acceptables.

2. Utilisation selon la revendication 1, dans laquelle les dérivés de la cystéine sont choisis notamment parmi l'acétylcystéine, la carboxyméthylcystéine, la méthylcystéine, l'éthylcystéine, la dansylcystéine, la carboxyéthylcystéine.

3. Utilisation selon la revendication 1, dans laquelle les dérivés de la cystine sont choisis notamment parmi la dansylcystine, la dinitrophénylcystine.

4. Utilisation selon l'une quelconque des revendications 1 à 3, dans laquelle le principe actif est présent sous la forme d'un sel d'un métal, notamment un sel de calcium, de sodium, de magnésium, de sélénium ou de potassium.

5. Utilisation selon l'une quelconque des revendications 1 à 3, dans laquelle le principe actif est présent sous la forme d'un sel d'une base organique, notamment de triéthylamine, de tris (hydroxyméthyl) amino méthane, d'éthanolamine.

6. Utilisation selon l'une quelconque des revendications 1 à 5, dans laquelle le principe actif est présent à une dose variant d'environ 30 mg à environ 4 000 mg, de préférence d'environ 100 mg à environ 1 000 mg.

7. Utilisation selon l'une quelconque des revendications 1 à 6, dans laquelle la composition comprend en outre de la vitamine B₆.

8. Utilisation selon l'une quelconque des revendications 1 à 7, dans laquelle la composition comprend en outre des excipients, des émulsifiants, des agents aromatisants et/ou des conservateurs.

9. Utilisation selon l'une quelconque des revendications 1 à 8, dans laquelle la composition est administrée par voie orale, rectale ou parentérale, notamment intraveineuse, intramusculaire ou sous-cutanée.

10. Utilisation selon l'une quelconque des revendications 1 à 9, dans laquelle la composition est sous forme de comprimé, pilule, poudre, granulé, sirop, suppositoire, solution injectable.

11. Utilisation selon l'une quelconque des revendications 1 à 10, en association avec un traitement médicamenteux responsable ou soupçonné responsable d'induire à titre d'effet secondaire une thrombopénie ou une thrombopathie.

12. Utilisation selon l'une quelconque des revendications 1 à 10, en tant qu'ingrédient supplémentaire d'une composition pharmaceutique responsable ou soupçonnée responsable d'induire à titre d'effet secondaire une thrombopénie ou une thrombopathie.
